# EUROPÄISCHE PATENTANMELDUNG

(11) **EP 1 811 043 A1**
(43) Veröffentlichungstag der Anmeldung: **25.07.2007**
(21) Anmeldenummer: 07100005.3
(22) Anmeldetag: 02.01.2007
(51) Int. Cl.: C12Q 1/68

(54) **Massenspektroskopische Analyse von Nukleinsäurefragmenten**

(30) Priorität: 24.01.2006 DE 102006003415
(71) Anmelder: SIEMENS AKTIENGESELLSCHAFT, 80333 München (DE)
(72) Erfinder: Mosner, Jörn, Dr., 91054 Erlangen (DE); Schmidt, Sebastian, Dr., 91058 Erlangen (DE)

(57) **Zusammenfassung**

Ein Verfahren zur Analyse einer Probe, wobei die Probe wenigstens ein Nukleinsäuremolekül mit wenigstens einer Nukleinsäuresequenz enthält, umfasst folgende Verfahrensschritte:
- Bereitstellen eines Vergleichsmoleküls bekannter Konzentration mit einer bekannten Vergleichssequenz, wobei die Vergleichssequenz im Vergleich zu einer Referenzsequenz der Nukleinsäuresequenz einen definierten Massenunterschied aufweist,
- Schneiden der Nukleinsäuresequenz zu Fragmenten der Nukleinsäuresequenz und der Vergleichssequenz zu Fragmenten der Vergleichssequenz,
- Ermitteln eines Massenspektrums einer Mischung der Fragmente der Nukleinsäuresequenz und der Vergleichssequenz mittels Massenspektrometrie,
- Ermitteln eines Vergleichsspektrums der Fragmenten der Vergleichssequenz mittels Massenspektrometrie,
- Bestimmung der Konzentration der Nukleinsäuresequenz aus dem Massenspektrums und dem Vergleichsspektrum und
- Ermitteln von noch nicht bekannten Sequenzvariationen der Nukleinsäuresequenz im Vergleich zur Referenzsequenz aus dem Massenspektrums und einem Referenzspektrum der Referenzsequenz.

## Beschreibung

Die vorliegende Erfindung betrifft ein Verfahren zur Analyse einer Probe, wobei die Probe wenigstens ein Nukleinsäuremolekül mit wenigstens einer Nukleinsäuresequenz enthält.

Die Bedeutung molekularbiologischer Analysen gewinnt in der Medizin zunehmend an Bedeutung. So lassen sich mit entsprechenden Methoden beispielsweise Erreger im Körper eines Patienten nachweisen und quantifizieren. Dabei wird der Erreger beispielsweise anhand charakteristischer DNA-Sequenzen identifiziert, so dass eine entsprechende Therapie für den Patienten erarbeitet werden kann. Insofern kommt der Bestimmung von DNA-Sequenzen und deren Nachweis eine zunehmende Bedeutung zu.

Gegenwärtig wird DNA-Sequenzierung entweder durch das chemische Abbauverfahren von Maxam und Gilbert (Methods in Enzymology 65, 499-560 (1980)) oder durch das enzymatische Didesoxynukleotid-Terminationsverfahren von Sanger et al. (Proc. Natl. Acad. Sci. USA 74, 5463-67 (1977)) durchgeführt. Im chemischen Verfahren führen basenspezifische Modifikationen zu einer basenspezifischen Spaltung des radioaktiv- oder fluoreszenzmarkierten DNA-Fragments. Mit den vier getrennten basenspezifischen Spaltungsreaktionen werden vier Sätze verschachtelter Fragmente hergestellt, die nach der Länge durch Polyacrylamid-Gelelektrophorese (PAGE) getrennt werden. Nach Autoradiographie kann die Sequenz direkt gelesen werden, da jede Bande (Fragment) im Gel aus einem basenspezifischen Spaltungsereignis herrührt. Daher werden die Fragmentlängen der vier "Leitern" direkt in eine spezifische Position in der DNA-Sequenz übersetzt.

Die Gelelektrophorese hat einige Nachteile. Die Herstellung eines homogenen Gels durch Polymerisierung, Laden der Proben, die Elektrophorese selbst, die Detektion des Sequenzierungsmusters (z. B. durch Autoradiographie), Entfernen des Gels und Saubermachen der Glasplatten, um ein anderes Gel herzustellen, sind sehr arbeitsaufwändige und zeitraubende Verfahren. Überdies ist das ganze Verfahren fehleranfällig, schwer zu automatisieren, und es wird ein ausgebildetes und geübtes Personal benötigt, um die Reproduzierbarkeit und Zuverlässigkeit zu verbessern. Im Falle einer radioaktiven Markierung kann die Autoradiographie selbst von Stunden bis Tagen dauern. Im Falle einer Fluoreszenzmarkierung kann wenigstens die Detektion der Sequenzierungsbanden automatisch durchgeführt werden, wenn Vorrichtungen zum Laserscannen verwendet werden, die in kommerziell verfügbare DNA-Sequenzierer integriert sind. Ein Problem, das mit der Fluoreszenzmarkierung verbunden ist, ist der Einfluss der vier verschiedenen basenspezifischen Fluoreszenz-Tags auf die Mobilität der Fragmente während der Elektrophorese und eine mögliche Überlappung bei der spektralen Bandbreite der vier spezifischen Farbstoffe, was die Unterscheidungskraft zwischen benachbarten Banden reduziert und dadurch die Wahrscheinlichkeit von Sequenzunklarheiten erhöht.

Im Allgemeinen stellt Massenspektrometrie ein Mittel zum "Wiegen" von individuellen Molekülen bereit, indem die Moleküle im Vakuum ionisiert werden und durch Verdampfen zum "Fliegen" gebracht werden. Unter dem Einfluss von Kombinationen von elektrischen und magnetischen Feldern folgen die Ionen Flugbahnen in Abhängigkeit von ihrer individuellen Masse und Ladung. Auf dem Gebiet von Molekülen mit niedriger Molekülmasse ist Massenspektrometrie seit langem ein Bestandteil des routinemäßigen physikalisch-organischen Repertoires zur Analyse und Charakterisierung organischer Moleküle durch die Bestimmung der Masse des Stammmolekülions. Zusätzlich wird, indem Kollisionen dieses Stammmolekülions mit anderen Partikeln (z.B. Argonatomen) veranlasst werden, das Molekülion durch die so genannte Kollisions-induzierte Dissoziation (CID) fragmentiert, wobei sekundäre Ionen gebildet werden. Das Fragmentierungsmuster bzw. der Fragmentierungsweg ermöglicht oftmals, detaillierte Strukturinformation abzuleiten.

Aufgrund der anscheinenden analytischen Vorteile von Massenspektrometrie bei der Bereitstellung einer hohen Detektionsempfindlichkeit, der Genauigkeit der Massemessungen und der Geschwindigkeit als auch einer Online-Datenübertragung auf einen Computer besteht erhebliches Interesse an der Verwendung von Massenspektrometrie für die Analyse von Nukleinsäuren.

Die Massenspektrometrie als Messmethode für die Größe der vervielfältigten DNA-Segmente hat beträchtliche Vorteile: es wird nicht die Ionenmobilität in Flüssigkeit oder polymerisiertem Gel gemessen, sondern direkt die Masse der Ionen. Die Ionenmobilität hängt in kritischer Weise von Formfaktoren ab und es kann daher vorkommen, dass die (manchmal zufällig angenommene) Faltungsstruktur der Moleküle eine Verfälschung der Messungen ergibt. Aber auch der (manchmal zufällig abweichende) Polymerisierungsgrad des Gels beeinflusst die Wanderungsgeschwindigkeit der DNA-Segmente, so dass es häufig zu Artefakten kommt.

Mit diesen Arten von Artefakten ist in der Massenspektrometrie nicht zu rechnen. Die Massenspektrometrie bietet darüber hinaus prinzipiell eine höhere Genauigkeit der Massenbestimmung. Aber auch die massenspektrometrische Messung der Massen der DNA-Segmente unterliegt Einschränkungen. Die Messung der DNA allgemein und insbesondere die Genauigkeit der Massenbestimmung wird besonders durch drei Effekte beeinflusst: (1) Adduktbildung durch ubiquitäre Na- oder K-Ionen, die sich in zufälligen Mengen an die DNA-Segmente anlagern, (2) die Bildung von Doppelpeaks durch verschiedene Massen der beiden DNA-Einzelstränge und (3) die Fragmentierung der zu messenden ssDNA-Segmente während des MALDI-Prozesses, meist durch Abspalten der Basen während der Ionisierung. Alle drei Effekte führen zu einer Verschmierung der Peaks: Adduktbildung zu höheren Massen hin, Fragmentierung zu niedrigeren Massen, und die Doppelpeakbildung zu einer oft nicht mehr als Doppelpeak erkennbaren Verbreiterung. Im hohen Massenbereich wirken diese Effekte so, dass nicht mehr auf ein Nukleotid genau gemessen werden kann.

Verfahren der Massenspektrometrie sind beispielsweise Elektrospray/Ionenspray (ESI), Ionen-Zyklotron-Resonanz (ICR) und matrixassistierte Laser-Desorption/Ionisation (MALDI). MALDI-Massenspektrometrie kann verbessert werden, wenn eine Flugzeit-(time-of-flight, TOF)-Konfiguration als Massenanalysator verwendet wird. Der gegenwärtig rasche Fortschritt in der MALDI-Technik führt zu hohem Automatisierungsgrad der Probenionisierung und zu kurzen Analysenzeiten pro Probe. Es werden damit Molekulargewichtsbestimmungen auch sehr großer Analytmoleküle in Mengen von einigen hundert Attomol in Messdauern von wenigen Sekunden möglich. Auf einem Probenträger können Hunderte von Proben untergebracht werden. Die Automatisierung und die hohe Dichte an Proben eröffnen mit der massenspektrometrischen Analyse die Möglichkeit zu einer massiv-parallelen Verarbeitung von einigen zehntausend Proben pro Tag.

MALDI ist ein Ionisationsverfahren für großmolekulare Analytsubstanzen auf Oberflächen. Die Analytsubstanzen werden zusammen mit geeigneten Matrixsubstanzen eines relativ zu den Analytmolekülen kleinen Molekulargewichts in Lösung auf eine Oberfläche gebracht, dort eingetrocknet und mit einem Laserpuls von wenigen Nanosekunden Dauer bestrahlt. Es verdampft eine geringe Menge an Matrixsubstanz, einige Moleküle davon als Ionen. Die in der Matrix sehr verdünnte Analytsubstanz, deren Moleküle in der Verdünnung vereinzelt sind, wird dabei mit verdampft, auch wenn ihr Dampfdruck normalerweise für eine Verdampfung nicht ausreicht. Die relativ kleinen Ionen der Matrixsubstanz reagieren mit den großen Molekülen der Analytsubstanz mit der Folge, dass die Analytsubstanzmoleküle durch Protonenübergang als Ionen zurückbleiben. Doppelsträngige DNA (dsDNA) wird dabei in der sauren Matrix zu einsträngiger DNA (ssDNA) reduziert.

Verfahren für die Sequenzierung der DNA nach dem Sanger-Schema unter Benutzung von PCR-Verfahren und Massenspektrometern mit MALDI- oder ESI-Technik sind bekannt, siehe dazu EP 0 679 196 B1.

Aus der US 6,238,871 B1 ist ein Verfahren bekannt, mittels dem sich eine Nukleinsäuresequenz ermitteln lässt. Darin wird die Nukleinsäuresequenz basenspezifisch geschnitten, so dass basenspezifisch endende Fragmente entstehen. Die Fragmente werden mittels Massenspektrometrie untersucht, wodurch ein Massenspektrum mit verschiedenen, zu den Fragmenten korrespondierenden Maxima entsteht. Durch Analyse des Massenspektrums ist die Sequenz der Nukleinsäure zu ermitteln. Dabei werden die Fragmentlänge und die damit in Zusammenhang stehenden Molekulargewichte berücksichtigt. Die beschriebene Methode basiert auf der Sanger-Sequenzierungsmethode und bietet den Vorteil, dass keine Gelelektrophorese oder ähnlich aufwändige Verfahren notwendig sind.

In der EP 0 815 261 B1 wird beschrieben, wie sich DNA-Sequenzen mittels Massenspektrometrie nachweisen lassen. Dazu werden Detektor-Olionukleotide mit einem oder mehreren Nukleinsäuremolekülen hybridisiert. Anschließend erfolgt eine Ionisierung und Verdampfung des hybridisierten Produktes. Die ionisierte und verdampfte Nukleinsäure wird mittels Massenspektrometrie analysiert, worin der Nachweis des Detektor-Olionukleotids durch Massenspektrometrie das Vorliegen der Target-Nukleinsäuresequenz in der Probe anzeigt. Im Allgemeinen wird vor der Hybridisierung das Nukleinsäuremolekül mit bekannten Amplifikationsverfahren, wie beispielsweise der Polymerase-Kettenreaktion (PCR), vervielfältigt. Zur Ermittlung der Massenspektren können verschiedene Verfahren zum Einsatz gelangen. Beispiele hierfür sind die Flugzeit-Massenspektrometrie, die matrixunterstützte Laser-Desorption/Ionisation und das Elektronensprühverfahren. Sollen mehrere Target-Nukleinsäuresequenzen nachgewiesen werden, so werden verschiedene Detektor-Oligonukleotide mit verschiedenen Massen an die Target-Nukleinsäuresequenzen hybridisiert. Auf diese Weise lassen sich im Massenspektrum die verschiedenen Target-Nukleinsäuresequenzen nachweisen.

PCR ist die gezielte Vervielfältigung eines genau ausgesuchten Stückes der zweisträngigen DNA. Die Auswahl des DNA-Segments erfolgt durch ein Paar von so genannten Primern, zweier ssDNA-Stücke mit je etwa 20 Basen Länge, die (etwas verkürzt und vereinfacht beschrieben) an beiden Seiten (den zukünftigen Enden) des ausgesuchten DNA-Stückes hybridisieren. Die Vervielfältigung erfolgt in einem einfachen Temperaturzyklus durch ein Enzym namens Polymerase, das eine chemische Fabrik in einem Molekül darstellt. Die PCR-Reaktion läuft in wässriger Lösung ab, in der wenige Moleküle der zu vervielfältigenden Ausgangs-DNA und genügende Mengen an Polymerase, Primern, Nukleinsäuren und Stabilisatoren vorhanden sind. In jedem Temperaturzyklus (beispielsweise Aufschmelzen der Doppelhelix bei 92°C, Hybridisieren der Primer bei 55°C, Wiedervervollständigung zu einer Doppelhelix durch Anbau neuer DNA-Glieder durch die Polymerase bei 72°C) wird das ausgewählte DNA-Segment im Prinzip verdoppelt. In 30 Zyklen werden also aus einem einzigen Doppelstrang der DNA als Ausgangsmaterial rund eine Milliarde DNA-Segmente erzeugt. (In strenger Beschreibung hybridisieren die beiden Primer auf den beiden verschiedenen Einzelsträngen der DNA und die Verkürzung auf das ausgewählte DNA-Segment von einem Primer zum anderen tritt erst statistisch bei weiterem Vervielfachen auf).

Die Polymerase kann unter optimalen Bedingungen etwa 500 bis 1000 Basen pro Sekunde an den Primer anbauen. Bei genügendem Überschuss an Primern, Polymerase und Substraten hängt die Zykluszeit praktisch nur von der Aufheiz- und Abkühlgeschwindigkeit ab, die wiederum vom Flüssigkeitsvolumen, Gefäßvolumen, Wärmeleitfähigkeit u.a. abhängt. Jeder Temperaturstufe ist im Prinzip nur wenige Sekunden zu halten.

Die Primer werden Teil der vervielfältigten DNA-Segmente, sie verbrauchen sich auf diese Weise während der PCR-Vervielfältigung. Dies bietet den Vorteil, dass sich beispielsweise chemische Gruppen (wie etwa fluoreszierende oder besonders gut ionisierende Gruppen) einbauen lassen, die für die spätere Detektion benutzt werden können. Die chemischen Gruppen werden zuvor an den künstlich hergestellten Primern angebaut.

Durch die Zugabe nur eines Primerpaares lassen sich uniforme DNA-Segmente vervielfachen. Werden andererseits gleichzeitig mehrere verschiedenartige Primerpaare zugegeben, so werden auch gleichzeitig mehrere uniforme DNA-Segmente vervielfältigt ("multiplexed PCR"). Diese Art der Multiplex-PCR wird häufig angewandt und hat oft besondere Vorteile. Für das so genannte "Fingerprinting" zur Identifizierung von Individuen durch DNA-Segmente variabler Länge (Methoden der "VNTR =Variable Number of Tandem Repeats" oder "AMP-FLP =Amplified Fragment Length Polymorphism") macht es die Analysen schneller. Dabei kann durch die Wahl der Primer, deren Abstand das mittlere Molekulargewicht der DNA-Segmente bestimmt, erreicht werden, dass die Variationen der Molekulargewichte der durch die verschiedenen Primerpaare gebildeten DNA-Segmente sich nicht oder nur selten überlappen. Diese Art der Multiplex-PCR bedingt einen Analysator, der zur gleichzeitigen Messung eines großen Bereichs der Molekulargewichte fähig ist.

Die genannten Messmethoden lassen sich beispielsweise in der Therapie chronischer Infektionen, wie z.B. mit dem HI-Virus einsetzen. Dabei wird regelmäßig zur Kontrolle des Therapieerfolgs und -verlaufs die Konzentration der Viren im Blut eines Patienten, die so genannte Viruslast bestimmt. Wird bei der Analyse eine Erhöhung der Viruslast im Vergleich zu vorangegangenen Untersuchungen festgestellt, kann auf eine Resistenzentwicklung des Virus gegen die in der Therapie verwendeten Medikamente geschlossen werden. Entsprechend ist bei einem derartigen Befund die Medikation entsprechend umzustellen.

Resistenzen des Virus beruhen im Allgemeinen auf einer Mutation in der DNA des Virus. Zur effektiven Umstellung der Medikation, ist es daher erforderlich, die DNA des Virus zu sequenzieren. Die ermittelte Sequenz des Gens wird mit bekannten Sequenzen verglichen, so dass Rückschlüsse auf Mutationen oder Sequenzvariationen geschlossen werden können, die zur Medikamentenresistenz führen.

Ein übliches Verfahren zur Bestimmung der Viruslast pro Blutvolumen besteht darin, das Virusgen und ein Vergleichsgen gleichzeitig zu amplifizieren und anschließend mittels optischer Detektion nachzuweisen. Aus dem Nachweis wird ein Konzentrationsverhältnis zwischen dem Virusgen und dem Vergleichsgen abgeleitet, so dass bei bekannter Konzentration des Vergleichsgens auf die Konzentration des Virusgens zurückgeschlossen werden kann. Dieses beschriebene Prinzip wird beispielsweise im System "Amplicor©" der Fa. Hofmann-La Roche verwendet.

Zur Ermittlung der Viruslast mittels der bereits beschriebenen Verfahren der Massenspektrometrie wird zunächst das zu analysierende Virusgen mit einem Vergleichsgen gemeinsam amplifiziert, beispielsweise mittels PCR oder "Primer Extension". Danach werden kurze Nukleinsäure-Fragmente durch Primer Extension erzeugt, deren Masse für das Virusgen und das Vergleichsgen unterschiedlich ist. Nach Bestimmung des Massenspektrums lassen sich durch die unterschiedlich gewählten Massen des Virus und des Vergleichsgens das Konzentrationsverhältnis von Virusgen und Vergleichsgen ermitteln. Durch Hinzunahme der bekannten Konzentration des Vergleichsgens lässt sich die Konzentration des Virusgens und damit des Virus in der Probe ermitteln.

Die Bestimmung der Gensequenz des Virusgens kann beispielsweise durch die Sequenzierung nach Sanger erfolgen. Dies ist beispielsweise im System "Trugene©" der Fa. Bayer der Fall, bei dem Gelelektrophorese zur Sequenzierung angewendet wird.

Zur Sequenzierung mittels Massenspektrometrie wird das Virusgen zunächst im Allgemeinen amplifiziert, wobei bereits modifizierte Basen eingebaut werden können, die ein spezifisches Schneiden erleichtern. Das amplifizierte Virusgen wird spezifisch an bestimmten Basen oder Sequenzmotiven geschnitten, beispielsweise durch Zugabe entsprechender Restriktionsenzyme. Die Massen der entstandenen Fragmente lassen sich mittels Massenspektrometrie ermitteln, woraus Rückschlüsse auf die Sequenz des untersuchten Gens möglich sind.

Oftmals werden beide Verfahren, also die Bestimmung der Viruslast und die Sequenzierung der Virus-DNA, nacheinander für denselben Patienten durchgeführt. So kann im Fall einer festgestellten, erhöhten Viruslast die weiterführende Therapie definiert und gezielt umgestellt werden. Beide Diagnosen werden bisher im Allgemeinen auf unterschiedlichen Geräten mit aufwändigen Verfahren durchgeführt, so dass dabei hohe Kosten für Personal und Verbrauchsmaterial entstehen. Zudem kommt es zu einer Verzögerung der diagnostischen Entscheidung, da die Verfahren im Allgemeinen zeitaufwändig sind. Da das Resistenzverhalten von bekannten Virusstämmen mit der Zeit größer wird und die Auswahl an Therapeutika zunimmt, kommt den entsprechenden Analysen künftig eine vermehrte Bedeutung zu.

In "Quantitative Analysis of Plasma TP53 249Ser-Mutated DANN by Electrospray Ionization Mass Spectrometry", Cancer Epidemiol Biomarkers Prev (2005) 14(12) 2956-2962 beschreiben M.E. LLeonart et al. Ein Verfahren, um die Konzentration und das Auftreten einer spezifischen Mutation mittels Massenspektrometrie zu ermitteln. Dabei werden interne Standards verwendet, die die gegenüber einem Wildtyp die gesuchte Mutation und eine gezielte Austauschbase enthalten. Auf diese Art lassen sich das Auftreten der 249^{Ser}-Mutation und deren Konzentration in Proben untersuchen.

Es ist Aufgabe der vorliegenden Erfindung, ein Verfahren zur Analyse einer Probe anzugeben, mittels dem sich sowohl eine Mutation als auch die Konzentration einer Nukleinsäure bestimmen lässt.

Diese Aufgabe wird durch ein Verfahren mit den Merkmalen des Anspruchs 1 gelöst. Alternativ wird diese Aufgabe durch ein Verfahren mit den Merkmalen des Anspruchs 2 gelöst.

Gemäß Anspruch 1 ein Verfahren zur Analyse einer Probe angegeben, wobei die Probe wenigstens ein Nukleinsäuremolekül mit wenigstens einer Nukleinsäuresequenz enthält, umfassend folgenden Verfahrensschritte:
- Bereitstellen eines Vergleichsmoleküls bekannter Konzentration mit einer bekannten Vergleichssequenz, wobei die Vergleichssequenz im Vergleich zu einer Referenzsequenz der Nukleinsäuresequenz einen definierten Massenunterschied aufweist,
- Schneiden der Nukleinsäuresequenz zu Fragmenten der Nukleinsäuresequenz und der Vergleichssequenz zu Fragmenten der Vergleichssequenz,
- Ermitteln eines Massenspektrums einer Mischung der Fragmente der Nukleinsäuresequenz und der Vergleichssequenz mittels Massenspektrometrie,
- Ermitteln eines Vergleichsspektrums der Fragmenten der Vergleichssequenz mittels Massenspektrometrie,
- Bestimmung der Konzentration der Nukleinsäuresequenz aus dem Massenspektrums und dem Vergleichsspektrum und
- Ermitteln von noch nicht bekannten Sequenzvariationen der Nukleinsäuresequenz im Vergleich zur Referenzsequenz aus dem Massenspektrums und einem Referenzspektrum der Referenzsequenz.

Das beschriebene Verfahren bietet den Vorteil, dass innerhalb einer einzelnen Analyse sowohl die Konzentration der zu bestimmenden Nukleinsäuresequenz als auch das Ermitteln von Sequenzvariation im Vergleich zu einer Referenzsequenz möglich ist. Durch das Einbringen der Vergleichssequenz mit definiertem Massenunterschied zur Nukleinsäuresequenz lässt sich innerhalb des Massenspektrums das Konzentrationsverhältnis des Vergleichsmoleküls und des Nukleinsäuremoleküls bestimmen. Wie bei bekannten Verfahren kann hierbei die Konzentration des Nukleinsäuremoleküls bestimmt werden. Dasselbe Massenspektrum kann auch zur Ermittlung von Sequenzvariationen verwendet werden, indem ein Vergleich mit einem Referenzspektrum der Referenzsequenz angestellt wird. Auf diese Weise lassen sich die ansonsten in getrennten Verfahren auszuführenden Analysen in einem einzigen Verfahren ausführen.

Das in Anspruch 2 beschriebene Verfahren zur Analyse einer Probe, wobei die Probe wenigstens ein Nukleinsäuremolekül mit wenigstens einer Nukleinsäuresequenz enthält, umfasst folgende Verfahrensschritte:
- Bereitstellen eines Vergleichsmoleküls bekannter Konzentration mit einer bekannten Vergleichssequenz, wobei die Vergleichssequenz im Vergleich zu einer Referenzsequenz der Nukleinsäuresequenz einen definierten Massenunterschied aufweist,
- gleichzeitige Amplifikation des Nukleinsäuremoleküls und des Vergleichsmoleküls,
- Schneiden eines Amplifikationsprodukts zu Fragmenten der Nukleinsäuresequenz und zu Fragmenten der Vergleichssequenz,
- Ermitteln eines Massenspektrums einer Mischung der Fragmente der Nukleinsäuresequenz und der Vergleichssequenz mittels Massenspektrometrie,
- Ermitteln eines Vergleichsspektrums der Fragmenten der Vergleichssequenz mittels Massenspektrometrie,
- Bestimmung der Konzentration der Nukleinsäuresequenz aus dem Massenspektrums und dem Vergleichsspektrum und
- Ermitteln von noch nicht bekannten Sequenzvariationen der Nukleinsäuresequenz im Vergleich zur Referenzsequenz aus dem Massenspektrums und einem Referenzspektrum der Referenzsequenz.

Das Verfahren bietet dieselben Vorteile, wie das Verfahren in Anspruch 1, es wird jedoch eine Amplifikation des Nukleinsäuremoleküls und des Vergleichsmoleküls durchgeführt. Dies ist insbesondere der Fall, wenn zu wenige Exemplare des Nukleinsäuremoleküls in der Probe vorliegen.

Die Amplifikation kann beispielsweise durch Klonieren, transkriptionsbasierte Amplifikationen, Polymerase-Kettenreaktionen (PCR), Ligase-Kettenreaktion (LCR) oder Strangumordnungsamplifikation (SDA) erfolgen.

In einer bevorzugten Ausführungsform der Erfindung basiert die Vergleichssequenz auf einer zur Nukleinsäuresequenz korrespondieren Referenzsequenz. Der Massenunterschied wird durch Ersetzen wenigstens einer, in der Vergleichssequenz auftretenden Ausgangsbase durch wenigstens eine Markierungsbase erzeugt. Dies hat den Vorteil, dass der auftretende Massenunterschied im Massenspektrum deutlich hervortritt und somit die Auswertung des Massenspektrums erleichtert wird.

In einem vorteilhaft ausgebildeten Verfahren erfolgt das Schneiden der Nukleinsäuresequenz und das Schneiden der Vergleichssequenz durch Zugabe wenigstens eines Restriktionsenzyms (Endonuklease). Die Verwendung von Restriktionsenzymen zum Schneiden von Nukleinsäuresequenzen ist bekannt. Es steht eine Vielzahl von Restriktionsenzymen zur Verfügung, die an spezifischen Basen die Nukleinsäure zerschneiden. Durch den bekannten Schnittpunkt lässt sich aus dem Massenspektrum und den darin ermittelten Molekulargewichten auf die Sequenz der Nukleinsäure schließen.

Besonders vorteilhaft ist ein Verfahren derart, dass die Markierungsbase und das Restriktionsenzym derart gewählt sind, dass die Nukleinsäuresequenz an der Ausgangsbase geschnitten wird und die Vergleichssequenz an der Markierungsbase nicht geschnitten wird. Dadurch entsteht in der Vergleichssequenz ein Fragment weniger durch das Schneiden mit dem Restriktionsenzym als in der Nukleinsäuresequenz. Folglich treten im Massenspektrum drei Maxima auf, die durch ihren Massenunterschied eindeutig identifizierbar sind.

Vorteilhaft ist ein Verfahren derart, dass die Bestimmung der Konzentration der Nukleinsäuresequenz folgende Verfahrensschritte umfasst:
- Analyse von im Massenspektrum auftretenden und zu den Fragmenten korrespondierenden Maxima,
- Identifizieren eines der auftretenden Maxima, das mit dem Fragment der Vergleichssequenz korrespondiert, das die Markierungsbase beinhaltet,
- Identifizieren zweier weiterer Maxima, die mit den beiden Fragmenten der Nukleinsäuresequenz korrespondieren, die durch das Schneiden der Nukleinsäuresequenz an der Ausgangsbase entstanden sind,
- Ermittlung eines Konzentrationsverhältnisses des Nukleinsäuremoleküls und des Vergleichsmoleküls durch Bestimmung des Flächenverhältnisses des einen Maximums zu der Summe der zwei weiteren Maxima und
- Ermittlung der Konzentration des Nukleinsäuremoleküls durch Division des Konzentrationsverhältnisses durch die Konzentration des Vergleichsmoleküls.

Durch das so erweiterte Verfahren lässt sich die Konzentration der Nukleinsäuresequenz auf einfache Weise ermitteln.

Ein weiteres vorteilhaftes Verfahren umfasst zum Ermitteln der Sequenzvariationen folgende Verfahrensschritte:
- Normierung des Vergleichsspektrums auf die Konzentration der Vergleichssequenz,
- Ermitteln eines Differenzspektrums durch Subtraktion des normierten Vergleichsspektrums vom Massenspektrum,
- Ermitteln der Sequenzvariationen durch Vergleich des Differenzspektrums mit dem Referenzspektrum.

So lassen sich aus demselben Massenspektrum, das bereits zum Ermitteln der Konzentration des Nukleinsäuremoleküls verwendet wurde, auch die auftretenden Sequenzvariationen ermitteln.

Weitere Vorteile der Erfindung ergeben sich anhand des nachfolgend beschriebenen Ausführungsbeispiels im Zusammenhang mit den beigefügten Zeichnungen. Es zeigen:
Figur 1 ein schematisches Ablaufdiagramm einer bevorzugten Ausführungsform der Erfindung,
Figuren 2 bis 5 verschiedene schematische Massenspektren während unterschiedlichen Verfahrensschritten.

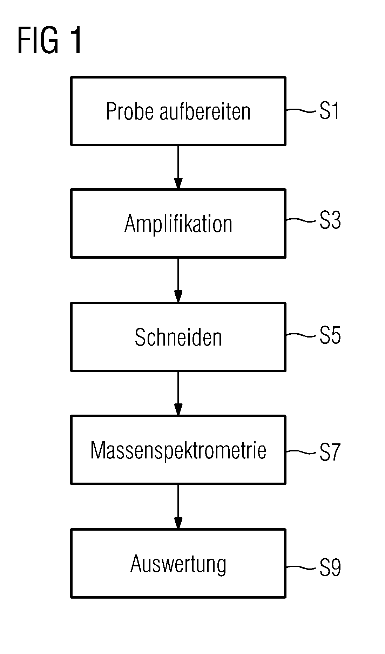

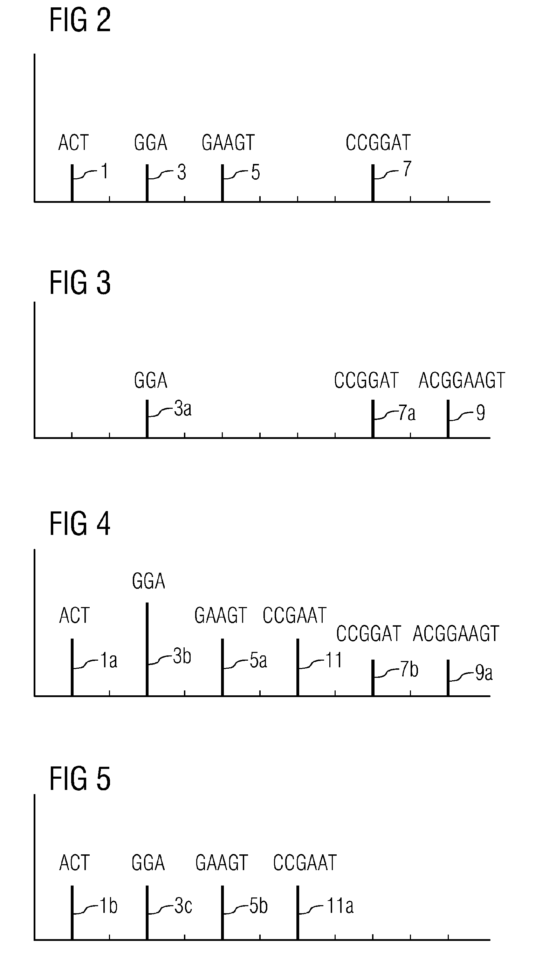

Anhand von Figur 1 soll zunächst der Verfahrensablauf zur Bestimmung der Konzentration und der Sequenz einer DNA beschrieben werden. Details zur Auswertung der ermittelten Massenspektren werden mit beispielhaften Massenspektren anhand der Figuren 2 bis 5 erläutert. Das beschriebene Verfahren kann für eine Vielzahl von Untersuchungen eingesetzt werden, beispielsweise in der Therapie von HIV-Erkrankungen oder Hepatitis.

Gemäß Figur 1 wird in einem ersten Verfahrensschritt S1 eine Probe entnommen und für die Analyse aufbereitet. So wird beispielsweise einem HIV-Patienten eine Blutprobe entnommen, aus der durch entsprechende Aufbereitung die Virus-RNA des HI-Virus extrahiert und dem weiteren Verfahren zur Verfügung gestellt wird. Dabei ist es zunächst erforderlich, die HI-Viren aufzukonzentrieren und durch Zellaufschluss die Virus-RNA aus den Viren zu extrahieren. Um eine Analyse vornehmen zu können, ist eine Umwandlung der Virus-RNA in eine korrespondierende cDNA-Sequenz erforderlich. Dies kann beispielsweise durch Zugabe Reverser Transkriptase erfolgen. Zur Reinigung und Isolation der RNA- bzw. DNA-Sequenz sind verschiedene Verfahren bekannt. So lassen sich beispielsweise mittels immobilisierten Fänger-Olionukleotiden die entsprechenden DNA-Sequenzen hybridisieren und durch Spülen mit einer Waschlösung reinigen. Durch Lösen der Hybridisierung lassen sich die entsprechenden DNA-Sequenzen weiter verarbeiten.

Zu der gereinigten und bereitgestellten DNA-Sequenz wird eine bekannte Vergleichssequenz gegeben, die der nachzuweisenden Nukleinsäuresequenz nahezu identisch ist. Sie entstammt einem entsprechenden Vergleichsmolekül und weist im Vergleich zur nachzuweisenden Nukleinsäuresequenz einen messbaren Massenunterschied auf. Die Vergleichssequenz wird dadurch erzeugt, dass in einer bekannten Referenzsequenz eines Referenzmoleküls, das dem zu analysierenden Nukleinsäuremolekül bis auf die noch nicht bekannten Sequenzvariationen identisch ist, eine Ausgangsbase durch eine Markierungsbase ersetzt wird. Dies wird anhand der Figuren 2 bis 5 detailliert erläutert.

Die zugefügte Vergleichssequenz bzw. das zugefügte Vergleichsmolekül wird mit dem Nukleinsäuremolekül zusammen in einem zweiten Verfahrensschritt S3 amplifiziert. Durch die Amplifikation werden die Nukleinsäuresequenz und die Vergleichssequenz gleichzeitig derart oft vervielfältigt, dass sie später mittels Massenspektrometrie nachweisbar sind. Die Amplifikation kann mit bekannten Verfahren, wie beispielsweise PCR erfolgen. Sollten ausreichende Exemplare der Nukleinsäuresequenz und der Vergleichssequenz in der Probe vorliegen, kann auf die Amplifikation auch verzichtet werden.

Nach der Amplifikation wird in einem dritten Verfahrensschritt S5 ein Restriktionsenzym zugesetzt, durch das das Amplifikationsprodukt, also die Nukleinsäuresequenz und die Vergleichssequenz, an basenspezifischen Stellen geschnitten werden. Das Restriktionsenzym ist dabei derart gewählt, dass die Vergleichssequenz an der Markierungsbase nicht geschnitten wird, aber die Nukleinsäuresequenz an der Ausgangsbase geschnitten wird.

Bei der Auswahl der Ausgangsbase, die in der Vergleichssequenz durch eine Markierungsbase ersetzt werden soll, ist es wichtig, einen Teil der Referenzsequenz zu wählen, der mit Sicherheit nicht durch eine relevante Sequenzvariation des Virusgenoms betroffen ist. Die Ausgangsbase kann also ohne Beeinflussung des Untersuchungsergebnisses der Sequenzierung durch die Markierungsbase ersetzt werden. Dazu wird eine Sequenz des Virusgenoms verwendet, die geringe Mutationsraten aufweist. Die Nukleinsäuresequenz ist beispielsweise aus früheren Untersuchungen bis auf die auftretenden und zu untersuchenden Sequenzvariationen bekannt. Im Vergleich zur bekannten Referenzsequenz ist es erforderlich, die Sequenzvariation zu ermitteln. Die Vergleichssequenz und somit das Vergleichsmolekül entstehen nun beispielsweise durch Synthetisierung des Referenzmoleküls, bei dem allerdings die Ausgangsbase gezielt durch die Markierungsbase ersetzt ist. Die Markierungsbase ist dabei derart gewählt, dass sie mit Sicherheit nicht von einer Sequenzvariation im noch unbekannten Nukleinsäuremolekül betroffen ist. In der Nukleinsäuresequenz des zu untersuchenden Nukleinsäuremoleküls liegt also mit hoher Wahrscheinlichkeit die Ausgangsbase vor, so dass sich die Vergleichssequenz an den Stellen der aufgetretenen Sequenzvariationen und an der Markierungsbase von der Nukleinsäuresequenz unterscheidet. Dies wird bei der Beschreibung der Figur 2 im Detail erläutert.

Durch das Schneiden der Nukleinsäuresequenz und der Vergleichssequenz entstehen basenspezifisch endende Fragmente. Durch die Auswahl der Markierungsbase und des Restriktionsenzyms entsteht bei der Nukleinsäure ein Fragment mehr als bei der Vergleichsnukleinsäure.

In einem vierten Verfahrensschritt S7 wird ein gemeinsames Massenspektrum der geschnittenen Nukleinsäuresequenz und der geschnittenen Vergleichssequenz ermittelt. In dem Massenspektrum treten Maxima auf, die den erzeugten Fragmenten der Nukleinsäuresequenz und der Vergleichssequenz entsprechen. In einem Großteil der Fragmente stimmen beide Nukleinsäuresequenzen überein. Lediglich die Fragmente, in denen Sequenzvariationen vorliegen und in dem Fragment, das durch die Markierungsbase verändert wurden, unterscheiden sich die Fragmente und damit die auftretenden Maxima. Da die Höhe der auftretenden Maxima von der Anzahl der vorliegenden Fragmente des jeweiligen Typs abhängt, treten folglich Maxima verschieden hoher Intensität auf.

Das Massenspektrum kann beispielsweise durch Flugzeit-Massenspektrometrie, matrixunterstützte Laser-Desorption/Ionisation (MALDI), Elektrospray-Ionisation (ESI), Ionenzyklotron-Resonanz (ICR) oder eine Kombination dieser Verfahren ermittelt werden.

Zusätzlich wird im vierten Verfahrensschritt ein Massenspektrum der Vergleichssequenz ermittelt. Dieses kann entweder durch eine separate Messung eines Massenspektrums erfolgen oder durch eine Simulation auf Basis der bekannten Vergleichssequenz.

In einem fünften Verfahrensschritt S9 erfolgt die Auswertung der ermittelten Massenspektren und die Bestimmung der Konzentration des Nukleinsäuremoleküls und damit des Krankheitserregers, sowie die Ermittlung von Sequenzvariationen der Nukleinsäuresequenz im Vergleich zur Referenz- und zur Vergleichssequenz. Die Auswertung wird detailliert anhand der Figuren 2 bis 5 erläutert.

In den Figuren 2 bis 5 sind verschiedene Massenspektren der beteiligten geschnittenen Nukleinsäuren schematisch dargestellt. Dabei sind lediglich die Lagen der relevanten Maxima durch Striche angedeutet, wobei die eigentlich gemessene Kurve aus Gründen der Übersichtlichkeit nicht dargestellt wird. Auf der horizontalen Achse ist in den Figuren 2 bis 5 eine Skala steigender Masse aufgetragen, während in der vertikalen Richtung die Intensität des gemessenen Spektrums aufgetragen ist.

Es wird im vorliegenden Ausführungsbeispiel davon ausgegangen, dass die bekannte Referenzsequenz folgende Sequenz aufweist:
ACTGAAGTCCGGATGGA

Dabei stehen die Buchstaben A, T, G und C für die vier Basen Adenin, Thymin, Guanin und Cytosin, aus denen Nukleinsäuresequenzen aufgebaut sind. Die Referenzsequenz ist beispielsweise aus einer vorhergehenden Sequenzierung bekannt. Als Vergleichssequenz wird folgende Sequenz verwendet:
ACGGAAGTCCGGATGGA.

Die Vergleichssequenz unterscheidet sich von der Referenzsequenz lediglich in der dritten Base, wobei in der Referenzsequenz Thymin als Ausgangsbase vorliegt, während in der Vergleichssequenz Guanin als Markierungsbase vorliegt. Die entsprechenden Fragmente der Vergleichs- und der Referenzsequenz weisen somit einen detektierbaren Massenunterschied auf. Die in Teilen noch unbekannte und zu analysierende Nukleinsäure soll im vorliegenden Ausführungsbeispiel folgende Sequenz aufweisen:
ACTGAAGTCCGAATGGA.

Die unbekannte Nukleinsäuresequenz unterscheidet sich lediglich in einer Mutation Referenzsequenz, es liegt folglich eine einzelne Sequenzvariation vor. Eine Guaninbase wurde dabei durch die hervorgehoben dargestellte Adeninbase ersetzt. Diese Mutation gilt es durch das vorliegende Verfahren zu ermitteln. Gleichzeitig ist zu ermitteln, in welcher Konzentration die Nukleinsäure in der entnommenen Blutprobe des Patienten vorliegt. Es ist zu beachten, dass die Base Thymin an dritter Position sowohl in der Referenz- als auch in der Nukleinsäuresequenz vorliegt.

Durch das verwendete Restriktionsenzym werden die vorliegenden Nukleinsäuresequenzen basenspezifisch hinter dem Thymin in einzelne Fragmente zerschnitten. So wird die Referenznukleinsäure beispielsweise hinter der dritten, der achten und der vierzehnten Base in folgende vier Fragmente zerschnitten.
ACT GAAGT CCGGAT GGA

In einem entsprechenden Massenspektrum der Referenznukleinsäure, das in Figur 2 dargestellt ist, treten folglich vier Maxima 1, 3, 5 und 7 auf. Das Maximum 1 korrespondiert zum Fragment ACT, das Maximum 3 zum Fragment GGA, das Maximum 5 zum Fragment GAAGT und das Maximum 7 zum Fragment CCGGAT.

Die Vergleichssequenz wird dahingegen lediglich an der achten und der vierzehnten Base geschnitten, so dass lediglich die drei folgenden Fragmente vorliegen:
ACGGAAGT CCGGAT GGA

Folglich treten auch im entsprechenden Massenspektrum, das schematisch in Figur 3 dargestellt ist, lediglich drei Maxima 3a, 7a und 9 auf. Die zwei Maxima 3a und 7a korrespondieren zu den Maxima 3 und 7 des in Figur 2 dargestellten Massenspektrums des Referenzmoleküls und damit zu den Fragmenten GGA bzw. CCGGAT. Die in der Figur 2 dargestellten Maxima 1 und 5 treten im Massenspektrum des Vergleichsmoleküls nicht auf, da diese zwei Fragmente aufgrund der ausgetauschten Markierungsbase nicht geschnitten wurden. Stattdessen tritt ein neues Maximum 9 auf, das mit dem Fragment ACGGAAGT korrespondiert und einen signifikant höheren Massenwert aufweist.

Das Nukleinsäuremolekül wird durch das Restriktionsenzym in folgende Fragmente geschnitten:
ACT GAAGT CCGAAT GGA

Diese entsprechen bis auf die Mutation den Fragmenten des Referenzmoleküls.

In Figur 4 ist das im vierten Verfahrensschritt S7 ermittelte Massenspektrum der Mischung der Nukleinsäuresequenz und der Vergleichssequenz schematisch dargestellt. Entsprechend den in der Mischung auftretenden Fragmenten erscheinen Maxima innerhalb des Massenspektrums. Es erscheint ein Maximum 1a (ACT), das zu Maximum 1 des in Figur 2 dargestellten Massenspektrums korrespondiert. Es entspricht dem Fragment, das an der Ausgangsbase abgeteilt wurde. Das auftretende Fragment 3b (GGA) entspricht der an der vierzehnten Base abgeteilten Fragment und tritt ebenfalls als Maximum 3 in Figur 2 und als Maximum 3a in Figur 3 auf. Das auftretende Maximum 5a entspricht dem Maximum 5 in Figur 2 und somit dem vor der achten Base abgeteilten Fragment GAATT. Dieses Fragment ist in Figur 3 nicht aufgetreten, da das Vergleichsmolekül nicht an der der Markierungsbase vom Restriktionsenzym geschnitten wurde. Des Weiteren treten die Maxima 7b (CCGGAT) und 9a (ACGGAAGT) auf, die den Maxima 7a und 9 aus Figur 3 entsprechen. Das Maximum 9a entspricht des durch die Markierungsbase nicht geteilten Fragments der Vergleichssequenz. Dieses tritt in der Nukleinsäuresequenz nicht auf, so dass zum Maximum 9a lediglich die Fragmente des Vergleichsmoleküls beitragen. Bei den vorliegenden Maxima 1a und 5a tragen nur die Fragmente der Nukleinsäuresequenz zur Intensität des Maximums bei, da sie in der Vergleichssequenz nicht auftreten. Zur Intensität des Maximums 3b tragen sowohl die Nukleinsäuresequenz als auch die Vergleichssequenz bei, da das entsprechende Fragment in beiden Nukleinsäuresequenzen vorkommt. Aufgrund der vorliegenden Sequenzvariationen im Vergleich zwischen dem Nukleinsäuremolekül und dem Vergleichsmolekül treten zwei Maxima 7b und 11 im Spektrum auf. Das Maximum 7b entspricht dem Maximum 7a aus Figur 3 und somit dem nicht von der Sequenzvariation betroffenen Fragment CCGGAT des Referenzmoleküls. Durch die Sequenzvariation tritt ein neues Maximum 11 im Spektrum auf, das mit dem Fragment CCGAAT korrespondiert.

Zur Bestimmung der Konzentration des Nukleinsäuremoleküls werden die Maxima 1a, 5a und 9a zur Auswertung herangezogen. Von diesen Maxima ist bekannt, zu welchen Fragmenten sie korrespondieren, da hier gezielt die Ausgangsbase durch die Markierungsbase ersetzt wurde. Die hier nicht dargestellte Fläche unter den Maxima 1a und 5a entsprechen in der Summe der relativen Konzentration des Nukleinsäuremoleküls. Allerdings ist aus der Fläche die absolute Konzentration nicht ohne weiteres zu ermitteln, da eine Referenz fehlt. Als Referenz wird die Fläche unter dem Maximum 9a verwendet, die der relativen Konzentration des Vergleichsmoleküls entspricht. Da das Vergleichsmolekül in definierter Konzentration zugegeben wurde, wird der Absolutwert für die Konzentration des Vergleichsmoleküls für eine Normierung der Flächen unter den Maxima verwendet. Dadurch lässt sich die absolute Konzentration des Nukleinsäuremoleküls ermitteln. Dies erfolgt durch Summierung der Flächen unter den Maxima 1a und 5a mit nachfolgender Division durch die Fläche des Maximums 9a. Anschließend wird mit der bekannten Konzentration des Vergleichsmoleküls multipliziert, woraus sich die Konzentration des Nukleinsäuremoleküls ergibt.

Zur Bestimmung der Sequenzvariation wird das in Figur 3 dargestellte Massenspektrum der Vergleichssequenz auf die Höhe des Maximums 7b bzw. 9a in der Figur 4 normiert. Das dargestellte Vergleichsspektrum kann durch eine Messung oder eine Simulation ermittelt werden. Anschließend wird das normierte Spektrum von dem in Figur 4 dargestellten Massenspektrum subtrahiert, so dass lediglich ein Massenspektrum der zu ermittelnden Nukleinsäuresequenz verbleibt. Durch die vorangegangene Normierung werden die Maxima der Vergleichssequenz vollständig entfernt. Ein entsprechendes Massenspektrum ist in Figur 5 schematisch dargestellt. Hier treten lediglich die Maxima 1b (ACT), 3c (GGA), 5b (GAAGT) und 11a (CCGAAT) auf. Durch Vergleich mit dem Massenspektrum des Referenzmoleküls aus Figur 2 lässt sich die Sequenzvariation ermitteln. Es wird festgestellt, dass anstatt des ursprünglichen Maximums 7 (CCGGAT) das verschobene Maximum 11a (CCGAAT) auftritt. Anhand der absoluten Massenverschiebung zwischen den Spektren und der Kenntnis der Sequenz des Fragments CCGGAT des Referenzmoleküls lässt sich feststellen, welche Sequenzvariation vorliegt.

Folglich lässt sich mit dem beschriebenen Verfahren die Konzentration beispielsweise von Erregern und eventuelle Sequenzvariationen ihrer DNA oder RNA ermitteln. Dabei sind die angewendeten Verfahren zur Sequenzierung bzw. Analyse von Sequenzvariationen mittels Massenspektrometrie bekannt. Es wird beispielsweise auf die eingangs genannten Literaturstellen verwiesen.

Alternativ ist es möglich eine Aliquotierung des Amplifikats oder der Probe vorzunehmen. Die Aliquots werden dann zur Primer Extension und zum basensepzifischen Schneiden verwendet und entsprechend dis Konzentration und die Sequenz der Virus-DNA bestimmt.

### Annex zu den Anmeldungsunterlagen - nachgereichtes Sequenzprotokoll

## Patentansprüche

1. Verfahren zur Analyse einer Probe, wobei die Probe wenigstens ein Nukleinsäuremolekül mit wenigstens einer Nukleinsäuresequenz enthält, umfassend folgende Verfahrensschritte:
- Bereitstellen eines Vergleichsmoleküls bekannter Konzentration mit einer bekannten Vergleichssequenz, wobei die Vergleichssequenz im Vergleich zu einer Referenzsequenz der Nukleinsäuresequenz einen definierten Massenunterschied aufweist,
- Schneiden der Nukleinsäuresequenz zu Fragmenten der Nukleinsäuresequenz und der Vergleichssequenz zu Fragmenten der Vergleichssequenz,
- Ermitteln eines Massenspektrums einer Mischung der Fragmente der Nukleinsäuresequenz und der Vergleichssequenz mittels Massenspektrometrie,
- Ermitteln eines Vergleichsspektrums der Fragmente der Vergleichssequenz mittels Massenspektrometrie,
- Bestimmung der Konzentration der Nukleinsäuresequenz aus dem Massenspektrum und dem Vergleichsspektrum und
- Ermitteln von noch nicht bekannten Sequenzvariationen der Nukleinsäuresequenz im Vergleich zur Referenzsequenz aus dem Massenspektrum und einem Referenzspektrum der Referenzsequenz.

2. Verfahren zur Analyse einer Probe, wobei die Probe wenigstens ein Nukleinsäuremolekül mit wenigstens einer Nukleinsäuresequenz enthält, umfassend folgenden Verfahrensschritte:
- Bereitstellen eines Vergleichsmoleküls bekannter Konzentration mit einer bekannten Vergleichssequenz, wobei die Vergleichssequenz im Vergleich zu einer Referenzsequenz der Nukleinsäuresequenz einen definierten Massenunterschied aufweist,
- gleichzeitige Amplifikation des Nukleinsäuremoleküls und des Vergleichsmoleküls,
- Schneiden eines Amplifikationsprodukts zu Fragmenten der Nukleinsäuresequenz und zu Fragmenten der Vergleichssequenz,
- Ermitteln eines Massenspektrums einer Mischung der Fragmente der Nukleinsäuresequenz und der Vergleichssequenz mittels Massenspektrometrie,
- Ermitteln eines Vergleichsspektrums der Fragmenten der Vergleichssequenz mittels Massenspektrometrie,
- Bestimmung der Konzentration der Nukleinsäuresequenz aus dem Massenspektrums und dem Vergleichsspektrum und
- Ermitteln von noch nicht bekannten Sequenzvariationen der Nukleinsäuresequenz im Vergleich zur Referenzsequenz aus dem Massenspektrums und einem Referenzspektrum der Referenzsequenz.

3. Verfahren nach Anspruch 2, worin die Amplifikation durch Klonieren, transkriptions-basierte Amplifikation, Polymerasekettenreaktion (PCR), Ligasekettenreaktion (LCR) oder Strangumordnungsamplifikation (SDA) erfolgt.

4. Verfahren nach Anspruch 1 oder 2, bei dem die Vergleichssequenz auf der zur Nukleinsäuresequenz korrespondierenden Referenzsequenz basiert und der Massenunterschied durch Ersetzen wenigstens einer, in der Referenzsequenz auftretenden Ausgangsbase durch wenigstens eine Markierungsbase in der Referenznukleinsäure erzeugt wird.

5. Verfahren nach Anspruch 1 oder 2, bei dem eine Sequenzierung der Nukleinsäuresequenz durch Analyse der im Massenspektrum auftretenden Molekulargewichte durchgeführt wird.

6. Verfahren nach Anspruch 1, bei dem das Schneiden der Nukleinsäuresequenz und das Schneiden der Vergleichssequenz durch Zugabe wenigstens eines Restriktionsenzyms erfolgen.

7. Verfahren nach Anspruch 1, bei dem die Markierungsbase und das Restriktionsenzym derart gewählt sind, dass die Nukleinsäuresequenz an der Ausgangsbase geschnitten und die Vergleichssequenz an der Markierungsbase nicht geschnitten wird.

8. Verfahren nach Anspruch 1, bei dem die Bestimmung der Konzentration der Nukleinsäuresequenz folgende Verfahrensschritte umfasst:
- Analyse von im Massenspektrum auftretenden und zu den Fragmenten korrespondierenden Maxima,
- Identifizieren eines der auftretenden Maxima, das mit dem Fragment der Vergleichssequenz korrespondiert, das die Markierungsbase beinhaltet,
- Identifizieren zweier weiterer Maxima, die mit den beiden Fragmenten der Nukleinsäuresequenz korrespondieren, die durch das Schneiden der Nukleinsäuresequenz an der Ausgangsbase entstanden sind,
- Ermittlung eines Konzentrationsverhältnisses des Nukleinsäuremoleküls und des Vergleichsmoleküls durch Bestimmung des Flächenverhältnisses des einen Maximums zu der Summe der zwei weiteren Maxima und
- Ermittlung der Konzentration des Nukleinsäuremoleküls durch Division des Konzentrationsverhältnisses durch die Konzentration des Vergleichsmoleküls.

9. Verfahren nach Anspruch 1, bei dem das Ermitteln der Sequenzvariationen folgende Verfahrensschritte umfasst:
- Anpassen des Vergleichsspektrums an die Konzentration der Vergleichssequenz,
- Ermitteln eines Differenzspektrums durch Subtraktion des angepassten Vergleichsspektrums vom Massenspektrum,
- Ermitteln der Sequenzvariationen durch Vergleich des Differenzspektrums mit dem Referenzspektrum.

10. Verfahren nach Anspruch 1, bei dem das Ermitteln des Massenspektrums und des Vergleichsspektrums durch Flugzeit-Massenspektrometrie, Matrix-unterstützte Laser-Desorption/Ionisation (MALDI), Elektrospray-Ionisation (ESI), Ionen-Zyklotron-Resonanz (ICR) oder eine Kombination dieser Verfahren erfolgt.

11. Verfahren nach Anspruch 1, bei dem das Ermitteln des Massenspektrums durch Flugzeit-Massenspektrometrie, Matrix-unterstützte Laser-Desorption/Ionisation (MALDI), Elektrospray-Ionisation (ESI), Ionen-Zyklotron-Resonanz (ICR) oder eine Kombination dieser Verfahren und das Ermitteln des Vergleichsspektrums durch eine Simulation erfolgt.

12. Verfahren nach Anspruch 1, bei dem das Nukleinsäuremolekül DNA ist.

13. Verfahren nach Anspruch 1, bei dem das Nukleinsäuremolekül RNA ist und vor der Amplifikation in eine korrespondierende cDNA-Sequenz umgewandelt wird.

14. Verfahren nach Anspruch 1, bei dem die Umwandlung durch Reverse Transkriptase erfolgt.

15. Verfahren nach Anspruch 1, bei dem das Nukleinsäuremolekül einem Krankheitserreger entstammt.

16. Verfahren nach Anspruch 1, bei dem das Nukleinsäuremolekül aus der Probe extrahiert wird.

17. Verfahren nach Anspruch 1, bei dem die Extraktion des Nukleinsäuremoleküls durch einen Zellaufschluss mit anschließender Immobilisierung des Nukleinsäuremoleküls und Waschen des Nukleinsäuremoleküls erfolgt.


SEQUENCE LISTING

<110>  Siemens AG

<120>  Verfahren zur Analyse einer Probe

<130>  2005P22114EP

<140>  07100005.3-2403
<141>  2007-01-02

<160>  11

<170>  PatentIn version 3.3

<210>  1
<211>  17
<212>  DNA
<213>  Artificial

<220>
<223>  Zur Veranschaulichung des Verfahrens

<400>  1
actgaagtcc ggatgga                                                    17


<210>  2
<211>  17
<212>  DNA
<213>  Artificial

<220>
<223>  Zur Veranschaulichung des Verfahrens

<400>  2
acggaagtcc ggatgga                                                    17


<210>  3
<211>  17
<212>  DNA
<213>  Artificial

<220>
<223>  Zur Veranschaulichung des Verfahrens

<400>  3
actgaagtcc gaatgga                                                    17


<210>  4
<211>  3
<212>  DNA
<213>  Artificial

<220>
<223>  Zur Veranschaulichung des Verfahrens

<400>  4
act                                                                    3


<210>  5
<211>  5
<212>  DNA
<213>  Artificial

<220>
<223>  Zur Veranschaulichung des Verfahrens

<400>  5
gaagt                                                                    5


<210>  6
<211>  6
<212>  DNA
<213>  Artificial

<220>
<223>  Zur Veranschaulichung des Verfahrens

<400>  6
ccggat                                                                   6


<210>  7
<211>  3
<212>  DNA
<213>  Artificial

<220>
<223>  Zur Veranschaulichung des Verfahrens

<400>  7
gga                                                                      3


<210>  8
<211>  8
<212>  DNA
<213>  Artificial

<220>
<223>  Zur Veranschaulichung des Verfahrens

<400>  8
acggaagt                                                                 8


<210>  9
<211>  6
<212>  DNA
<213>  Artificial

<220>
<223>  Zur Veranschaulichung des Verfahrens

<400>  9
ccggat                                                                   6


<210>  10
<211>  3
<212>  DNA
<213>  Artificial

<220>
<223>  Zur Veranschaulichung des Verfahrens

<400>  10
gga                                                                      3


<210>  11

<211>  6
<212>  DNA
<213>  Artificial

<220>
<223>  Zur Veranschaulichung des Verfahrens

<400>  11
ccgaat                                                                 6